# EUROPEAN PATENT SPECIFICATION

(11) **EP 1 593 745 B1**
(45) Date of publication and mention of the grant of the patent: **14.02.2007**
(21) Application number: 04447112.6
(22) Date of filing: 04.05.2004
(51) Int. Cl.: C12Q 1/68

(54) **Customized micro-array construction and its use for target molecule detection**
Personalisierte Mikroarrays and ihre Verwendung zum Nachweis von Zielmolekülen
Micro-réseaux personnalisés et leur utilisation pour la détection des molécules ciblées

(43) Date of publication of application: 09.11.2005
(73) Proprietor: EPPENDORF AG, 22331 Hamburg (DE)
(72) Inventor: Remacle, José, 5020 Malonne (BE); Beyreuther, Konrad, 69120 Heidelberg (DE); Peters, Lars-Erik, Lafayette, Colorado 80026 (US)
(74) Representative: Van Malderen, Joëlle

(56) References cited:
- WO-A-02/072889
- US-A- 5 700 637
- US-A1- 2003 003 495
- US-A1- 2004 009 506
- DE LONGUEVILLE F. ET AL.: "Gene expression profiling of drug metabolism and toxicology markers using a low-density DNA microarray" BIOCHEMICAL PHARMACOLOGY, vol. 64, no. 1, 1 July 2002 (2002-07-01), pages 137-149, XP002297537 ISSN: 0006-2952
- GUO Z. ET AL.: "DIRECT FLUORESCENCE ANALYSIS OF GENETIC POLYMORPHISMS BY HYBRIDIZATION WITH OLIGONUCLEOTIDE ARRAYS ON GLASS SUPPORTS" NUCLEIC ACIDS RESEARCH, vol. 22, no. 24, 1994, pages 5456-5465, XP008018942 ISSN: 0305-1048
- SCHENA M. ET AL.: "PARALLEL HUMAN GENOME ANALYSIS: MICROARRAY-BASED EXPRESSION MONITORING OF 1000 GENES" PROCEEDINGS OF THE NATIONAL ACADEMY OF SCIENCES OF USA, vol. 93, no. 20, 1 October 1996 (1996-10-01), pages 10614-10619, XP002022507 ISSN: 0027-8424
- SCHENA M. ET AL.: "Microarrays: biotechnology's discovery platform for functional genomics" TRENDS IN BIOTECHNOLOGY, ELSEVIER PUBLICATIONS, CAMBRIDGE, GB, vol. 16, no. 7, 1 July 1998 (1998-07-01), pages 301-306, XP004145643 ISSN: 0167-7799
- DUGGAN D.J. ET AL.: "EXPRESSION PROFILING USING CDNA MICROARRAYS" NATURE GENETICS, NEW YORK, NY, US, vol. 21, no. SUPPL, January 1999 (1999-01), pages 10-14, XP000865980 ISSN: 1061-4036
- YUEN T. ET AL.: "Accuracy and calibration of commercial oligonucleotide and custom cDNA microarrays" NUCLEIC ACIDS RESEARCH, OXFORD UNIVERSITY PRESS, SURREY, GB, vol. 30, no. 10, 2002, pages 1-9, XP002963922 ISSN: 0305-1048

## Description

### Field of the invention

The present invention is related to a method and kit of detection, identification and/or quantification of multiple target molecules possibly present simultaneously in a sample.

### Background of the invention and state of the art

Identification of multiple expressed genes, of organisms or of micro-organisms can be based on the presence in their genetic material of specific sequences. Identification and quantification of expressed genes is usually performed after reverse transcribing mRNA into its corresponding cDNA and detection of said cDNA via specific capture molecules present on or attached to micro-arrays. Detection of specific organisms can be performed easily by amplifying a particular sequence of their genomic DNA and then detecting and/or identifying these amplified sequences. Quantification of the target sequences bound to their specific capture molecules allows estimation of the amount of target molecules present in the initial sample. Preferably, appropriate control means are included and the necessary corrections made to take into account the efficiency of the different steps, such as the copying or amplification of target sequences and their capture via hybridization upon a micro-array.

Micro-arrays bearing arrays of nucleotide sequences are being produced mainly according to two methods. The first method, described in US patents 5, 510, 270 and 5,700,637, is based on photolithographic in situ synthesis of capture molecules or sequences on a solid support. Photolithographic DNA synthesis uses rapid solid phase phosphoramidite chemistry. Positional and sequential control are achieved by a combination of 5'-photoprotected phosphoramidites, which can be activated by irradiation with light, and a set of masks containing holes at appropriate positions through which light can pass. Upon excitation, the photoprotecting groups present on partial oligonucleotide sequences, synthesized earlier during the process, are removed and the oligomers are extended by another nucleotide after adding the relevant monomer. Current coupling efficiencies impose an actual size limit of about 25 bases to these chips. Beyond this limit, incomplete products accumulate.

The photolithographic method results in the presence of short oligonucleotides on a support. Using this methodology, a gene or a gene sequence is identified by a series of capture sequences of the same species rather than by a unique sequence. To be able to control specific hybridization of a particular target sequence, it is necessary to perform for each sequence a control, the control sequence being identical to the initial sequence but for one base difference. The main advantage of the method is the possibility to miniaturize the thus obtained arrays or chips and to generate high-density arrays containing several thousands of capture sequences.

The second method is based on the chemical or enzymatic synthesis of capture sequences before mechanical deposition onto known specific locations of the array substrate. With this method, there is no restriction in the size of the sequences to be spotted, deposited or attached. One major advantage of the deposition technology, compared to the *in situ* synthesis approach, is its great versatility. This method allows production of micro-arrays or chips for virtually any molecule of interest including but not limited to nucleic acid sequences of any length, antibodies, lipids, carbohydrates, small chemical compounds, etc. Furthermore, the synthesis of sequences can be optimized, sequences can be purified, their quality checked before use and/or their concentration adjusted before coupling to the solid surface. However, one disadvantage of the method is that the process is time-consuming since each sequence has to be handled separately before spotting on the micro-array or chip, thereby limiting the size of the arrays.

The chemistry of a hybridization-to-oligonucleotide micro-array is clearly different from that of an array constructed with long DNA capture sequences or molecules. It has been observed that long specific capture sequences give much better binding of a complementary target sequence present in a solution or sample than their corresponding short fragments. In practice long polynucleotide capture sequences are used for direct binding of long polynucleotide target molecules or sequences. In a typical gene expression experiment, the capture sequences for cDNA binding contain 50 bases or more, for example 70 bases, or may even contain 600 bases or nucleotides.

When short oligonucleotides of 15-20 bases only are used as capture sequences (see e.g. US 5, 510, 270) adequate detection, identification and/or quantification of long cDNAs is possible provided some modifications to the detection protocol. The RNA is first reverse transcribed into its corresponding cDNA by using a primer carrying a transcription start site for T7 RNA polymerase. This cDNA is then retranscribed *in vitro* into several RNA copies which are then cut into small pieces. These small RNA fragments are then used for hybridization on arrays bearing a series of capture sequences for each of these RNA fragments. Fragmentation is necessary to ensure sufficient access of the target RNA sequences to the very short capture sequences. Specific algorithms are required to adequately correlate the hybridization pattern of these different capture molecules with the original sequence(s) of the target DNA or mRNA.

A similar adaptation is made for the detection of double stranded DNA (dsDNA), which will preferentially re-associate in solution rather than being hybridized on capture sequences present on or attached to a solid substrate. Again the amplicons have to be retranscribed into RNA using a double amplification process performed with primer(s) bearing T3 or T7 sequences and then a retrotranscription with a RNA polymerase. These RNAs are cut into pieces of about 40 bases before being detected on an array (see e.g. example 1 of international patent application WO97/29212). The above technique was herein applied for the identification of the *Mycobacterium tuberculosis rpoB* gene, using capture nucleotide sequences of less than 30 nucleotides. The described method is complicated in the sense that it does not allow direct detection of amplicons resulting from genetic amplification reactions (such as PCR), but requires another cycle of reactions and copying of target sequences, which each introduce extra bias in the quantification of said target sequences.

Despite some disadvantages, the construction of micro-arrays via chemical synthesis and deposition of oligonucleotides or short polynucleotide sequences, is useful, since it is a fast and low-price process. In addition to that, the design of capture molecules or sequences can be easily adapted according to the requirements of the sequences to be analyzed or discriminated.

Capture molecules not necessarily have to consist of nucleotide sequences. Target molecules may as well be antibodies, antigen, receptors or ligands to be detected or captured by binding to their respective corresponding counterparts (antibody, antigen, ligand, receptor, ...). The above list of examples is non-exhaustive.

A customer is not always served with standard micro-arrays even though these may allow detection of a large amount of different target molecules. He may want to detect target molecules that are not custom, or may want to refine the level of discrimination. There is thus an ever increasing demand for customized or semi-customized micro-arrays to which the customer can add, at his wish, some extra detection molecules. The basic or standard micro-array that is further customized possibly already contains many different capture molecules for well determined gene detection.

Standard micro-arrays can be constructed and delivered to many users which can then use them for detection of some well defined target genes beside other target genes which would change from one application to the other or from one user to the other.

The US patent application US 2003/003495 discloses a detection and/or quantification method of target molecule biological samples by using micro-arrays comprising a set of immobilized capture molecules as well as adaptor molecules that can fix the target molecules to the capture molecules.

### Aims of the invention

The present invention aims to provide novel detection, identification and quantification methods and kit with standard micro-arrays that can be easily adapted for the specific needs of one or more particular customers for the detection of multiple target molecule

Another aim of the present invention is to allow a customer to detect in one particular experiment one set of new target molecules possibly changing from one experiment to the other beside a set of target molecules which are compatible with the capture molecules already bound to the surface of the solid support according to a micro-array, said detection of target molecules in both group being quantitative and related to their presence in the sample.

### Summary of the invention

The present invention is related to a method of detection or quantification of different sets of target molecules (a first set and a second set of target molecules) being possibly present simultaneously in a biological sample; said method comprises the step of putting into contact these sets of target molecules with a (micro)array present on a solid support surface; said (micro)array comprising a first and a second set of capture molecules, present in different locations of the solid support surface.
In the method of the invention, the first set of capture molecules comprises at least 3 capture molecules which are specific of the first set of target molecules, and which fix directly the first set of target molecules by specific molecular recognition (preferably by hybridisation), and the second set of capture molecules comprises at least 3 capture molecules which are unrelated and have no direct affinity with the second set of target molecules (and with the first set of target molecules); the second set of capture molecules is able to fix the second set of target molecules through an adaptor molecule which allows a complementary binding between capture molecules and target molecules.
Furthermore, in the method of the invention a relative quantification of one set of target molecules compared to the other set of target molecules is obtained advantageously by a correction factor calculated through the use of at least one additional identical target molecule (able to bind first set of capture molecules and second set through an adaptor molecule) simultaneously quantified on both capture molecules sets.
The correction factor used in the method according to the invention could be obtained by the person skilled in the art by using a target of unknown concentration which is able to fix the two sets of capture molecules or by using internal standard having a known concentration added to the sample and submitted to the same pre-treatment (purification, amplification, copy) steps and the contact step with the target molecules to be detected and/or quantified. Said international standard of known concentration is also able to bind with the two sets of capture molecules.
Therefore, in order to allow a suitable quantification of the target present in one of the two sets of target molecules, the person skilled in the art can identify the ratio between the two signals resulting from binding upon two capture molecules in the two sets, said signal is obtained in order to allow the person skilled in the art to provide a correction factor used for the quantification of the other target molecules to be detected and quantified.

Another aspect of the present invention, is related to a detection and a quantification kit which comprises:
- a (micro)array set solid support surface comprising a first and a second set of capture molecules present in different locations of the support surface, wherein the first set of capture molecule comprises at least three different capture molecules being specific to the target molecules to be detected and quantified in a sample, said capture molecules of the first set being able to fix directly a first set of target molecules;
- and wherein the (micro)array solid support surface further comprises at different locations of the solid support surface, a second set of capture molecules comprising at least three capture molecules unrelated and having no direct binding affinity to a second set of target molecules (and also the first set of target molecules) to be detected and quantified and possibly present simultaneously in the sample with the first set of target molecules;
- one or more adaptor molecule(s) which allow specific binding of the target molecules of the second set onto the capture molecules of the second set and;
- a correction factor for one set of target molecules that allows to compare the amount of target molecules of the first set with the target molecules of the second set being in the sample.

Preferably, in the method and kit according to the invention, the capture molecules of the second set are totipotent capture molecules ("universal micro array" portion). Therefore, they are unrelated and having no direct binding affinity to complementary target molecules by their own, said binding being allowed only through adaptor molecules.

According to a first embodiment of the present invention, the detection and quantification is performed on the (micro)array, divided into at least two different arrays, presented on the same surface of the solid support. Each array comprises first and second set of capture molecules present in different locations of the support surface. Said at least two different arrays, comprising capture molecules of the first set for the detection of the same target molecules and each other different array comprising different second sets of capture molecules for the detection of other target molecules, being different from one array to another. This means that it is possible to obtain a specific similar detection on a first location of target molecules, and different detection pattern of other types of target molecules following binding of said target molecules upon capture molecules of different arrays through specific adaptor molecules. Said adaptor molecules being different from one array to another array.

According to a second embodiment of the present invention, the fixing of the target molecules upon the second set of capture molecules is performed by a consumer addition of adaptor molecules upon the solid support. This means that the arrays according to the invention are "semi-customised" arrays which could be used for obtaining a specific binding according to the request of the consumer for specific identifications of target molecules at specific locations of the micro-array surface.

According to a first aspect of the present invention, the target, capture and the adaptor molecules are or comprise nucleotide sequences. In said embodiment, the fixing of the target molecules upon the second set of capture molecules is therefore obtained by a sandwich hybridization between the capture molecules and the target molecules through adaptor molecules being or comprising also complementary nucleotide sequences.

Preferably said sandwich hybridization is obtained by adaptor molecules which comprise a first portion, which allows a hybridization by complementary base pairing with a specific terminal portion of the capture molecules and a second portion, which is specific for at least a portion of one or more target molecules.

The specific terminal portion of a capture molecule being a nucleotide sequence, means the 5' or 3' terminal portion which is not bound to the surface of the solid support.

According to another aspect of the present invention, the target and capture molecules are proteins and adaptor molecules are chimeric proteins or hybrid antibodies which allows a specific indirect binding between capture and target molecules. According to a preferred example, the target and capture molecules are respectively
- either antigens and antibodies (or hypervariable portions of said antibodies).
- or antibodies (or portions) and antigens

The invention also provides means to determine (to quantify) the amounts of the two sets of target molecules detected in the initial sample. The amount of the different target molecules is either relative to each other or is corrected into absolute values (pmoles).
Quantitative detection is corrected by a factor which allows comparable and simultaneously detection of all fixed target molecules (fixed to their capture molecules). More preferably, in the method according to the invention, the quantitative detection is corrected by a detection of at least one standard sequence(s) added to the (biological) sample and submitted to the same detection steps as target molecules. Said standard molecule(s) are detected on both sets of capture molecules present on the two locations of the solid support surface.

In another aspect of the invention, the fixation efficiency of target molecules on the two groups of capture molecules is corrected by a quantitative detection of at least one common target in both sets. In a more specific embodiment, the common target molecule is a housekeeping gene.
Preferably, 3 standards or 3 target molecules are detected and quantified on both sets of capture molecules, each of the target molecules being different in concentration by at least a factor 3.

In order to allow a specific binding between capture molecules and target molecules, through adaptor molecules the capture molecules of the second set comprise one or more terminal reactive chemical function(s) able to bind specifically to the adaptor molecules. Preferably, said terminal reactive chemical function is selected from the group consisting of aldehyde groups, epoxy groups, acrylate groups or a mixture thereof.

Preferably all capture molecules are attached to the solid support surface by a covalent bond or link to form a micro-array on the solid support surface. In a first embodiment of the invention, the second location of the surface contains capture molecules terminated by a reactive chemical function (such as an aldehyde group, an epoxide group or an acrylate group that may react with a NH2 group of the adaptor molecule.

To avoid sterical hindrance, the capture molecules are bound to the surface of the solid support through a spacer (or link) of a particular length. This spacer element or molecule may be a polymeric chain of at least 10 atoms, possibly branched and selected from the group consisting of poly-ethyleneglycol, polyaminoacids, polyacrylamides, poly-aminosaccharides, polyglucides, polyamides, polyacrylates, polycarbonates, polyepoxides, poly-ester molecules or chains, or a mixture thereof. Alternatively, part of the capture molecule may act as a spacer molecule. Preferably, the spacer molecule is at least 6.8 nm long. Possibly, the spacer molecule is a nucleotide sequence of between about 15 and about 1000 bases, preferably between about 30 and about 120 bases.

The method and kit of the invention are particularly suited for the simultaneous detection, identification and/or quantification of biological molecules of interest such as target polynucleotides or nucleotide sequences (possibly homologous sequences simultaneously present in a biological sample or test solution), wherein a basic multi-parametric micro-array is adapted according to a customer's needs. Preferably, the target molecules such as target polynucleotides or nucleotide sequences (e.g. genomic DNA or RNA sequences or amplicons), prior to detection, are multiplied (copy) and/or amplified (genetic amplification) with techniques that are standard in the art. Alternatively, the detection signal may be amplified with techniques well known to a person skilled in the art.

In the context of the present invention, the meaning of the terms "nucleic acid", "oligonucleotide", "array" or "micro-array", "nucleotide sequence", "target nucleic acid" or "target nucleotide sequences", "bind substantially", "hybridizing specifically to", "background", "quantifying" and the like is as described in the international patent application WO97/27317.
The meaning of the term "homologous sequence(s)" is as described in European patent EP 1266034, which is incorporated by reference herein.

The term "solid support" for building micro-arrays in the present context is meant to comprise any type of solid material which can be physically handled to perform the necessary reactions like incubation of a test solution or sample possibly containing target molecules or copies of a target sequence. This solid support can be unique or can be a composite made of several materials, one of them being a substrate surface on which capture molecules or nucleotide sequences can be fixed or bound to yield a micro-array. Typical examples of substrates used in the field are polymers which may be functionalized (including submitted to the addition of a linker to their surface) in order to better fix the capture molecules. Said substrates or support surfaces on which the capture molecules are fixed or bound may be a porous or non porous support, may be smooth or rough and may be deposed or placed on top of another solid support, being made for example of glass or of plastic.

The terms "sample", "biological sample" or "test solution" are meant to comprise any (biological) sample or solution suspected to contain a target molecule. Said target molecule may be a (micro)organism or obtained from a (micro)organism or a component thereof (such as chloroplasts, mitochondries, genes, gene products, proteins, peptide, toxins, antigens, lipids, saccharides...)
In the context of the present invention, especially polynucleotides or nucleotide sequences specific to a (micro) organism or to a component (meat, bones, ...) thereof are being detected by the second set of capture molecules wherein target molecules are captured by a specific adaptor molecule. A test solution may be a solution containing PCR products (amplicon) of a (micro)organism or a component thereof.

In the present context the term "target molecule" may refer to a nucleotide sequence or polynucleotide, but also to a protein, a lipid, a saccharide etc. to be detected in a sample.

In the present context, a "capture molecule" may comprise a nucleotide sequence, but may as well comprise an antibody or hypervariable portion thereof (Fab, Fab2, etc.), an antigen or epitope thereof, a receptor, a ligand of a receptor, ... specific for a target molecule and/or specific for an adaptor molecule.

In the context of the present invention, the term "adaptor" may refer to a polynucleotide sequence or polypeptide sequence (such as an antibody, chimeric molecule, etc.) which makes the link between a capture molecule and a target molecule.

The invention will be described in further details in the following examples and specific embodiments, by reference to the enclosed drawings. The examples, embodiments and drawings are not in any way intended to limit the scope of the invention as claimed, neither are the reference signs used.

### Brief description of the figures

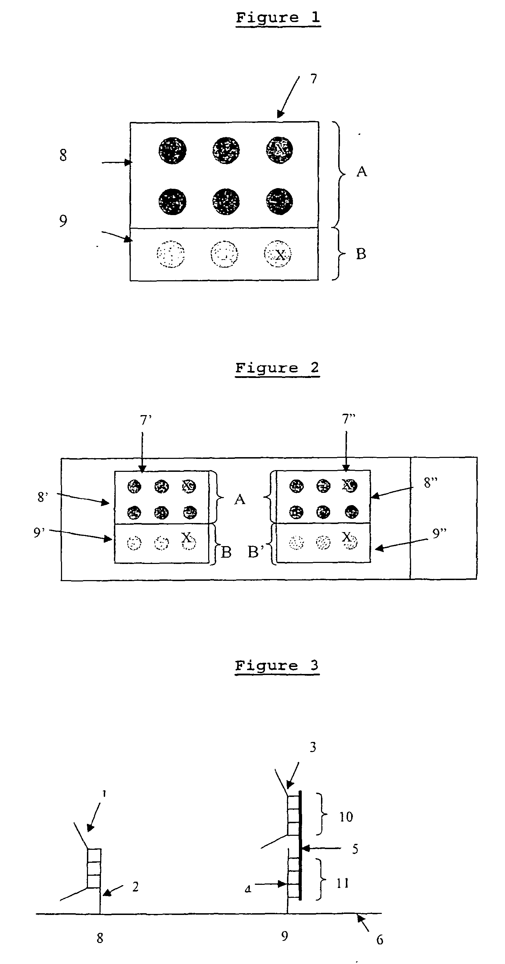

The figure 1 represents the surface of the solid support (6) in the form of an array (7), made of two sets of capture molecules (A, B) present in different locations (8, 9) of the solid support surface. The first set (A) is made of capture molecules (2) which are specific for the target molecules (1) whereas the second set (B) is made of capture molecules (4), unrelated to target molecules (3). One identical molecule (x) is simultaneously quantified on both sets (A, B) of capture molecules.

The figure 2 represents the surface of a glass slide covered with two "semi-customized" arrays (7', 7") according to the invention. In the first locations (8', 8"), the first set of capture molecules (A) is common on both arrays and they allow the detection of target molecules of the first group (1). However, on the second locations (9', 9"), the second sets of capture molecules (B, B') are different on both arrays and they allow the detection of different target molecules of the second group. One identical molecule (x) is simultaneously quantified on both sets (A, B-B') of capture molecules.
The figure 3 represents a solid support surface (6) according to the invention covered by two sets of capture molecules (2, 4) present in different locations of the solid support surface (8, 9). The first set of capture molecules (2) are specific for a first group of target molecules (1), while the second set of capture molecules is unrelated to a second group of target molecules (3), but can detect it through the use of a target (3) specific adaptor (5).

### Detailed description of the invention

The present invention is related to a method and kit which allows a detection and quantification of multiple target molecules (1, 3). Possible presented simultaneously in a sample by the detection after the fixation upon complementary capture molecules, present upon a solid support surface (6) according to an array (7).

Said surface (6) comprises at least two sets of capture molecules (2, 4), presented in different locations (8, 9) of the solid support surface (6), wherein a first set A comprising at least 3 different capture molecules (2) being specific for target molecules (1) to be detected, identified and/or quantified, said capture molecules being able to fix directly the target molecule 1; and wherein the second set B comprising at least 3 capture molecules (4), unrelated to other target molecules (3) to be detected and quantified and having no different affinity to fix the said target molecules (3), said second set of capture molecules (4) fixing the target molecules (3) through adaptor molecules (5). At least one identical molecule (x) is simultaneously quantified on both sets of capture molecules.

According to a preferred embodiment of the present invention described in the figure 1 and 3, the capture molecules are presented on the solid support surface 6 in the form of an array (7, 7', 7"). As described in the figure 2, the detection can be performed by using a solid support surface, comprising at least two different micro-arrays (7', 7") present on the surface (6) of said solid support; Said at least two arrays (7', 7") comprising capture molecules (2) of set A on first location (8', 8") for the detection of the same target molecules (1).

Furthermore, each array (7', 7") comprising also different sets (B, B') of capture molecules (4, 4') for the detection of target molecules (3, 3') being different from one array (7') to another (7"), said second set (B, B') of capture molecules (4, 4') being present on the different locations (9', 9") from the locations (8', 8") of the first set A.

According to the invention, this standard (micro-)array is adapted to detect, identify and/or quantify less common (other) target molecules (3) by adding to said standard support surface (6), that contains already capture molecules (2) for at least a first set of target molecules (1), adaptor molecules (5) that can bind to totipotent capture molecules (4), which can not directly bind to any of the target molecules (3) possibly present in a sample or a test solution. Said totipotent capture molecules (4) are already present (bound to the surface (6), on the standard micro-array (7) to customize. Preferably, said (totipotent) capture molecules (4) are nucleotide sequences such as DNA or RNA sequences, possibly with modified backbone.

Most preferably, attachment (fixation) to the support surface (6) of capture molecules (4) from the second set (B) does not lead to the complete detachment and/or inactivation of said first set (A) of capture molecules (2). Preferably said detachment and/or inactivation are kept as low as possible.

The standard micro-array or array (7) of the invention, prior to customization, thus contains two types of capture molecules (2, 4). The first type of capture molecules (2) is specific for a first group of target molecules (1), whereas the second type of capture molecules (4) are not specific for target sequences (1 or 3). They are totipotent, but can be made specific through binding of target-specific adaptor molecules (5).

In a preferred embodiment of the invention, totipotent capture molecules (4), adaptor molecules (5) and target molecules (3) are nucleotide sequences. Advantageously, the adaptor molecules (5) are specific to at least one part of the target polynucleotide (3) or of its copy, to allow its specific hybridization, detection, identification and/or quantification by indirect fixing upon capture molecules (4).

The preformed standard assays to be customized, also referred to as standard products (e.g. general chips, micro-arrays for the detection of cancer, mouse micro-arrays etc), are micro-arrays with capture molecules for the detection of a given number of well defined target molecules. The density of the capture nucleotide sequences bound to the solid support surface at a specific location is superior to about 10 fmoles, preferably about 100 fmoles per cm² of solid support surface. The standard products may be low density or high density arrays. Low density arrays make use of a standard product with a limited number of capture molecules for a limited and selected number of target molecules. The micro-arrays and the standard product of the invention contain at least 5, 10, 50, 100, 1000 or 10000 spots of capture molecules per cm².

The micro-arrays of the invention can be adapted for the detection of additional target molecules at the request of the user, in conjunction with target molecules detected by the first set of capture molecules. The same array according to the invention can thus be used for the detection of a large number of different target molecules, the number of possible target molecules to be detected being at least 10, 20, 50, 100 or even 500 times larger than the number of capture molecules present.

The present invention further extends to tools and reagents needed to customize the standard product and needed for subsequent detection, identification and/or quantification. The present invention is for instance also related to the above-described adaptor molecules (5) that are used to secure recognition and specific binding of target molecules (3) (possibly present in a biological sample or test solution), to the second set (B) of capture molecules (4) that are not able to bind directly to the said target molecules (3) present in the sample.

The second set (B) of capture molecules (4) do not have a nucleotide sequence complementary to a nucleotide stretch of a target molecule (3) to be detected or its complement.

In normal working conditions these target molecules (3), being polynucleotides or nucleotide sequences, will not bind to the capture molecules (4) if preferably less than 10 subsequent complementary bases are present or normally less than 15 complementary bases are present. Preferably there will be less than 5, more preferably less than 3, complementary base pairings possible between the target molecule and the totipotent capture molecule. Most preferably, no complementary base pair binding at all should be possible between a target polynucleotide or nucleotide sequence and this totipotent capture molecule. Hybridization, as known by the person skilled in the art, is defined by the percentage of identity or similarity of two sequences and is further defined by the hybridization conditions used (e.g. stringent, less stringent or non-stringent conditions), being dependent mostly of the temperature and the salt concentration.

An embodiment of the invention concerns customizable micro-arrays and their construction, starting from one and the same standard product, to be used for the detection of, on one hand, identical and identified target molecules and, on the other hand, of non-identified and variable target molecules.

In an embodiment of the invention, the above-described adaptor molecules (5) contain a sequence (10) of at least 30 consecutive nucleotides complementary to the target molecule (3) to be identified or to its complement, also referred to as the target-specific part of the adaptor molecule, and a sequence (11) of at least 30 consecutive nucleotides specific for the totipotent capture molecules (4) on which the target molecules will be detected and/or quantified indirectly, also referred to as the target non-specific or target-unrelated part of the adaptor molecule.

In another embodiment, the target-specific parts (10) of the adaptor molecule will consist of nucleotide sequences of more than 50 or 70 bases and even more than 100 or 150 bases.

In a preferred embodiment of the invention, the portion of the capture molecule (4) that is recognized by the adaptor molecule (5) exists at the terminal end (the extremity which is not bound to the surface (6) of the solid support) of said capture molecule (4) or is at least close to this end. It is preferably not too close to the other part or extremity of the capture molecule (4), through which the capture molecule (4) is attached to the solid support surface (6).

In another embodiment of the invention, the totipotent capture molecule (4) contains a sequence recognized by the adaptor molecule (5) and a sequence not recognized by the adaptor molecule (5) and not recognized by a target molecule (3). Preferably the latter is a spacer sequence of at least about 20 or 30 bases, preferably at least about 50 or 70 bases, more preferably at least about 100 or 150 bases long or a sequence comprised between said parameters.

In a preferred embodiment, the indirect binding of target molecules, via specific adaptor molecules (5) bound to totipotent capture molecules (4), will be with the same efficiency as the direct binding on the specific capture molecules (2) of the first set (A) present on the standard micro-array product (7). In another embodiment of the invention, the amount of adaptor molecule (5) added in the sample solution is adjusted in order to provide an efficiency of binding of target molecules (1, 3) that is then identical or similar for both sets (A, B), i.e. for the direct and for the indirect binding of the target molecules (1, 3).

In another embodiment, the quantification of a target molecule (1, 3) being present in a sample will be performed with a correction through the use of internal specific known standard sequences that are added in a specific known amount to the sample. Some of these sequences molecules will be detected by direct binding on the first set (A) of capture molecules (2), while other sequences will be detected indirectly trough the use of the above-described adaptor molecules (5), both types of binding possibly being corrected by a coefficient that is different for the two sets (A, B).

In a particular embodiment of the invention, the same internal standard will be captured both in the first set (A) of capture molecules by direct hybridization and in the second set of totipotent capture molecules by the use of adaptor molecules (5).

In another embodiment, the coefficient of correction for efficiency of hybridization for both sets of target molecules is obtained by the use of at least 1 and preferably at least 3 common target molecules being detected in both sets of capture molecules. In still another preferred embodiment, the 3 common target molecules are chosen in the high, medium and low copy number of genes present in the sample. The difference in the relative amount of such genes being at least a factor of 3 and preferably being 10 or more.
In one embodiment, the correction factor is different from one molecule to the other of the same set. The correction factor is common for a subset of molecules and is different for another subset of molecules of the same set. In a particular embodiment the molecules are classified into different subset according to their concentration and the factor of correction applied is different.

Quantification of target molecule like genes or their product (mRNA or protein) present in a sample is best performed using 3 different scanning settings in the array scanner (e.g. for the Packard array scanner settings used for the photomultiplier are 50, 70 and 100). The use of 3 settings and 3 targets or 3 internal standards present at different amounts, allows correction for efficiency of binding of both sets of target molecules in the 3 different scanning settings.

According to an embodiment of the invention, the efficiency of indirect binding of target molecules (3) on capture molecules (4) through adaptor molecules (5) is similar or identical to the direct binding of target molecules (1) on capture molecules (2) of at least about 100 or 150, more preferably at least about 200 or 300 and more preferably at least about 400 or 500 bases long.

In an embodiment of the invention, the micro-array contains capture molecules (2) of at least about 100 or 150 bases, more preferably at least about 200 or 300 bases and most preferably at least about 400 or 500 bases long for the direct capture of target molecules (1) to be detected in a biological sample or test solution.

In another embodiment, the capture molecules of both sets (A, B) are composed of a non-specific or unrelated sequence (spacer) being at least about 20 or 30 bases long, preferably at least about 50 or 70 bases long and still more preferably about 100 or 150 bases long, being attached to the support surface, and further at their terminal end a sequence specific either for the target molecule (1) or target sequence to be detected (in the case of direct binding) or for the adaptor molecule (5) (in the case of indirect binding). The specific or adaptor-specific part (11) of the capture molecules is at least 10 or 15 bases long, more preferably more than about 20 or 30 and even more preferably more than about 40 or 50 bases long. The length of the specific sequence of the capture nucleotide sequences (2) able to hybridize with the corresponding target nucleotide sequences may be comprised between about 10 and about 60 bases, more preferably between about 20 and about 30 bases. "About" in this context means that it is possible to have 1, 2, 3 to up to 5 nucleotides more or less than being mentioned.

In a particular embodiment of the invention, the capture molecules of the second set (B) are synthesized by PCR, using as template a common sequence. One primer has a floating sequence which differs from one capture sequence to the other and is used for the recognition of the adaptor molecule (5).

The arrays or micro-arrays (7,7',7") of the invention are highly suited for detection and/or quantification of genes expressed by cells, possibly after copying the target gene sequences at least in part into a cDNA strand and hybridization of the cDNA onto specific capture molecules provided according to the invention.

The method of the invention is particularly suited for the detection of sequences which are homologous to each other. Discrimination between homologous or nearly identical sequences is possible via the use of small or short specific sequences. For instance, the target-specific part of the capture molecules of the first group (A) or of the adaptor molecule (5), used to discriminate homologous target nucleotide sequences (1), may be between about 15 and about 50 bases long. The percentage of homology is preferably higher than about 40% or 50%, preferably higher than about 60%, 65% or 70%, more preferably higher than about 75%, 80%, 85% or 90%.

The method of the invention may even allow discrimination between target sequences that differ in one or a few nucleotides only and that thus contain one or a few SNPs (single nucleotide polymorphisms).

Advantageously, the method and arrays of the invention can be used for the identification and/or quantification of DNA sequences, single or double stranded sequences, either obtained directly from an organism or part thereof - i.e. without any prior amplification step - or, alternatively, after amplification of part of its genomic DNA or RNA. Amplification of target sequences may be achieved via any method known in the art, including but not limited to PCR, LCR, NASBA, rolling circle or any other method which allows the production of a rather reliable copy of the given sequence. One or more (PCR) primers or primer sets may be used in the genetic amplification procedure. Primers may be universal primers, consensus primers and/or type-specific primers. Possibly, a kit according to the invention may comprise chambers wherein the detection for the presence of any amplified sequences of (micro)organisms and the genetic amplification are performed in one and the same chamber. The means for a genetic amplification of target nucleotide sequences obtained from said (micro) organisms may be provided with the kit of the invention.

The amplified nucleotide sequence may be mRNA that is first retrotranscribed into cDNA, possibly using the same primer pair for retrotranscription and amplification.

Possibly, the presence of any amplified sequence is firstly detected during the genetic amplification cycles and thereafter identified on the array.

Detection of the target molecules captured on the micro-array can be done via any physical or chemical detection method known in the art including but not limited to the use of fluorescence marking, colorimetry, bioluminescence, chemiluminescence, electric, surface plasmon resonance, electromagnetic signals, .... Reading and interpretation of the capture or hybridization signal is enhanced by the fact that the capture molecules are linked to well-defined locations of the solid support.

The (insoluble) solid support or the substrate for the construction of the micro-array according to the invention preferably is selected from the group consisting of glasses, electronic devices, silicon supports, silica, metals or mixture thereof prepared in a format selected from the group of slides, discs, gel layers and/or beads. Beads are considered as arrays for as long as they have characteristics which allow to differentiate them from each other, so that identification of the beads is correlated with the identification of a given capture molecule and so of the target sequence. The above examples are not exhaustive.

In an embodiment of the invention, detection, identification and/or quantification of captured target sequences is facilitated by the use of an apparatus comprising at least a detection and/or quantification device to detect and/or quantify a signal formed at the location where a target molecule or component from a (micro)organism is captured on the array, possibly a reading device for information recorded upon a surface of said solid support, a computer program for recognizing the discrete regions bearing the bound target molecules upon its corresponding capture molecules and their locations, possibly a quantification program of the signal present at the locations and a program for correlating the presence of the signal at these location with the diagnostic and/or the quantification of the said (micro)organism or component. The present invention extends to an apparatus adapted for these purposes and able to read the micro-array of the invention and interpret its results.

The present invention further extends to the use of a customized array in a kit or detection method for the detection, identification and/or quantification of a mixture of biological molecules from a first and second set of target molecules. These target molecules may belong to different groups, sub-groups or sub-sub-groups of components or (micro)organisms. In an embodiment of the invention, the first set of capture molecules may be specific for individual target components or their sub-groups, whereas the second group is then specific for all the components of the group or vice versa.

The method and kit of the invention may be used for the detection of any kind of (micro)organism or any component thereof. Examples of (micro)organisms and components thereof include but are not limited to *Staphylococci* species selected from the group consisting of S. *aureus,* S. *epidermidis,* S. *saprophyticus, S. hominis* and/or S. *haemolyticus,* samples belonging to the *Mycobacteria* genus, sequences which belongs to the MAGE family or the *HLA-A* family, G coupled receptors, dopamine receptors, choline receptors, histamine receptors, members of the cytochrome P450 family, GRAM+ or GRAM- family bacteria.

One may target groups, sub-groups or individual target molecules or target sequences corresponding to families, genus, species, subtypes or individual organisms. Preferably, the families, genus, species, subtypes or individuals are bacteria such as those selected from the groups consisting of *Staphylococcus, Enterococcus, Streptococcus, Haemolyticus, Pseudomonas, Campylobacter, Enterobacter, Neisseria, Proteus, Salmonella, Simonsiella, Riemerella, Escherichia, Neisseria, Meningococcus, Moraxella, Kingella, Chromobacterium and Branhamella.* The above list is not-exhaustive.

### Examples

### Example 1: Construction of a micro-array having target-specific capture molecules and target-non specific capture molecules and detection of target molecules through the use of a specific adaptor molecule of 60 bases

### Functionalization of the glass support

The glass slides are functionalized for the presence of aldehydes according to the method described in European patent application EP1184349.

### Fixation of target-specific (set A) and target-non specific capture molecules (set B-) on the support

The specific capture molecules are directed against 59 genes as described by Delongueville et al 2002 (Biochem. Pharmacol. **64**:137-149). They target 59 toxicologically relevant genes in the Rat Hepatochip dualchip (Eppendorf, Hamburg, Germany). Three target-unrelated capture molecules are synthesized and spotted on the array. These 3 capture molecules are synthesized by PCR amplification from a cloned sequence of plant inserted into a plasmid. The 3 target molecules which are fixed through an adaptor on theses target unrelated capture molecules are simultaneously fixed on 3 target specific capture molecules. Since 3 target molecules are simultaneously quantified on both capture molecule sets, they will allow correction for the quantification of the target molecules.

The PCR sense primers that were used are the following:
PALFAS1 (SEQ ID NO: 1) for sequence 1:
   5'-gcatggatgttgctctcgcgtagacgactggatggctagttactgctctg-3'
PALUCP21 (SEQ ID NO: 2) for sequence 2:
   5'-agtacgtcgacagacttagtcctgaagctcgatggctagttactgctctg-3'
PALL191 (SEQ ID NO: 3) for sequence 3:
   5'-ggctgatcatgtactccaaggtttgttatcgatggctagttactgctctg-3'
The sense primers comprise 30 floating bases at the 5' end (bold) followed by 20 bases specific for the insert.

The antisense primer was common for the 3 PCR reactions. It is aminated at the 5' end and is specific for the insert.
PAL2 (SEQ ID NO: 4):
   5'-Amine-atgagtttcaagatttcaacag-3'

The sequences of the 3 capture molecules are the following:
TPALFAS (SEQ ID NO: 5):
   5'Amine-atgagtttcaagatttcaacatgagtttcaagatttcaacagcttctgatgttttcctt gaagagattaagcccaaggagtttacatcttgattgtgttgttcagcactttgaacatg gttggtcactggattggctaaaaattgaagttcagagcagtaactagccatccagagca gtaactagccatccagtcgtctacgcgagagcaacatccatgc-3'
TPALUCP2 (SEQ ID NO: 6):
   5'Amine-atgagtttcaagatttcaacatgagtttcaagatttcaacagcttctgatgttttcctt gaagagattaagcccaaggagtttacatcttgattgtgttgttcagcactttgaacatg gttggtcactggattggctaaaaattgaagttcagagcagtaactagccatccagagca gtaactagccatcgagcttcaggactaagtctgtcgacgtact-3'
TPALL19 (SEQ ID NO: 7):
   5'Amine-atgagtttcaagatttcaacatgagtttcaagatttcaacagcttctgatgttttcctt gaagagattaagcccaaggagtttacatcttgattgtgttgttcagcactttgaacatg gttggtcactggattggctaaaaattgaagttcagagcagtaactagccatccagagca gtaactagccatcgataacaaaccttggagtacatgatcagcc-3'

A negative control consist of a capture molecule of identical length comprising a 30 bases random sequence which is not recognized by an adaptor present in the solution.

The capture molecules are diluted in spotting solution (EAT Namur, Belgium). The two sets of capture molecules are spotted with an arrayer using plain pins on spatially separated locations of the support. The slides are dried 1h at room temperature. The slides are washed 2 times 2 min with washing buffer and 3 times with water. The slides are kept at 4°C until use.

### Detection of target cDNA on a customized micro-array of the invention

The surface of the support containing the capture molecules of the first and second sets are surrounded with a hybridization frame which delimits the surface of the support being in contact with the solution containing the target molecules. The array is first incubated for 30 min at 55°C with 3 different synthetic adaptor molecules. The adaptor molecules are polynucleotides of about 60 bases. They contain 30 bases complementary to the capture molecules and 30 bases complementary to the target cDNA sequences. The 3 target cDNA are FAS (Accession N°: U03470), UCP2 (Accession N°: AB010743) and L19 (Accession N°: J02650).

The sequences of the 3 adaptor molecules are the following:
AFAS30 (SEQ ID N0:8) :
   5'-ataaagttttgggctgctgtgtggcaatgcgcatggatgttgctctcgcgtagacgact g-3'
AUCP230 (SEQ ID NO:9):
   5'-atgccattgtcaactgtactgagctggtgaagtacgtcgacagacttagtcctgaagct c-3'
AL1930 (SEQ ID NO:10):
   5'-cgtcctccgctgtggtaaaaagaaggtgtgggctgatcatgtactccaaggtttgttat c-3'
The sequences complementary to the capture molecules are indicated in bold.

The adaptor molecules are obtained by chemical synthesis (Eurogentec, Liege, Belgium). After incubation, the array is washed with SSC2X solution. The array is then incubated with biotinylated cDNA from rat liver as explained here above (Delongueville et al. 2002). After hybridization with the biotinylated target cDNA, the arrays are incubated with anti-Cyanine antibody (Jackson ImmunoResearch, Cy3: ref.-200.162.096, Cy5: ref.-200.172.096) diluted 1/1000 in B1 buffer containing 0.01% blocking agent. The biochips are then washed 4 times during 2 min with B1 buffer. The biochips are dried at room temperature and scanned with GMS418 ScanArray (General Scanning). After digitalization of the picture, the imagene software (Biodiscovery, Marina Del Rey, USA) is used in order to delimitate the spot surface, integrate the signal for each spot, subtract the local background around each spot, identify the localization of the spots and correlate the localization with the identity of the target. The quantification of the target present in the sample is obtained essentially as described here above (Delongueville et al. 2002). The quantification of target molecules from both sets are corrected in order to determine their amount in the initial sample used in the analysis. Correction is obtained through the use of the 3 common target cDNAs (FAS, UCP2 and L19 described above) simultaneously quantified on both capture molecule sets.

### Example 2: Construction of a micro-array having target-specific capture molecules and target-non specific capture molecules and detection of target molecules through the use of a specific adaptor molecule of 80 bases

The experiment is performed as in example 1. The adaptor molecules used for the detection of the 3 target cDNA are polynucleotides of 80 bases. They contain 30 bases complementary to the capture molecules (bold) and 50 bases complementary to the target cDNA sequences.

The sequences of the 3 adaptor molecules are the following:
AFAS50 (SEQ ID NO: 11):
   5'-ataaagttttgggctgctgtgtggcaatgcagaggcaaagagaaggaactgcatggatg ttgctctcgcgtagacgactg-3'
AUCP250 (SEQ ID NO:12):
   5'-atgccattgtcaactgtactgagctggtgacctatgacctcatcaaagatagtacgtcg acagacttagtcctgaagctc-3'
AL1950 (SEQ ID NO:13):
   5'-cgtcctccgctgtggtaaaaagaaggtgtggttggaccccaatgaaaccaggctgatca tgtactccaaggtttgttatc-3'

### Example 3: Construction of a micro-array having target-specific capture molecules and target-non specific capture molecules and detection of target molecules and internal standards through the use of a specific adaptor molecule of 60 bases

The experiment is performed as in example 1 with the detection of 3 common target molecules on both sets of capture molecules. The micro-array also contains 3 internal-standard specific capture molecules in set A (RBCL, CAB10B, rubisco activase). Three additional target-unrelated capture molecules are used for correction of hybridization efficiency. They are used to capture adaptor molecules partially complementary to the internal standard cDNA.

These 3 new target-unrelated capture molecules are synthesized by PCR amplification from a cloned sequence of plant inserted into a plasmid. The floating sequences of 30 bases at the 5' end of the primers differ from example 1.

The following sense primers were used:
PALRBCL1 (SEQ ID NO:14) for sequence 1:
   5'-tctgttcccttacgactcgataagagctctgatggctagttactgctctg-3'
PALCAB1 (SEQ ID NO:15) for sequence 2:
   5'-actcttgctgaggctattcaaggcggcatcgatggctagttactgctctg-3'
PALRUBI1 (SEQ ID NO:16) for sequence 3:
   5'-atacagttcaatcgccgagtctacgcggtagatggctagttactgctctg-3'

Again, the sense primers comprise 30 floating bases at the 5' end (bold) followed by 20 bases specific for the insert.

The antisense primer is the same as in example 1 (SEQ ID NO:4).

The sequences of the 3 capture molecules used for the 3 internal standards are the following:
TPALRBCL (SEQ ID NO: 17):
   5'Amine-atgagtttcaagatttcaacatgagtttcaagatttcaacagcttctgatgttttcctt gaagagattaagcccaaggagtttacatcttgattgtgttgttcagcactttgaacatg gttggtcactggattggctaaaaattgaagttcagagcagtaactagccatccagagca gtaactagccatcagagctcttatcgagtcgtaagggaacaga-3'
TPALCAB (SEQ ID NO:18):
   5'Amine-atgagtttcaagatttcaacatgagtttcaagatttcaacagcttctgatgttttcctt gaagagattaagcccaaggagtttacatcttgattgtgttgttcagcactttgaacatg gttggtcactggattggctaaaaattgaagttcagagcagtaactagccatccagagca gtaactagccatcgatgccgccttgaatagcctcagcaagagt-3'
TPALRUBI (SEQ ID NO:19):
   5'Amine-atgagtttcaagatttcaacatgagtttcaagatttcaacagcttctgatgttttcctt gaagagattaagcccaaggagtttacatcttgattgtgttgttcagcactttgaacatg gttggtcactggattggctaaaaattgaagttcagagcagtaactagccatccagagca gtaactagccatctaccgcgtagactcggcgattgaactgtat-3'

The 3 internal standard cDNA are RBCL (Accession N°: L14403, incorporated by reference herein), CAB10B (Accession N°: M32606, incorporated by reference herein) and *Lycopersicon pennellii* rubisco activase (Accession N°: AF037361, incorporated by reference herein).

The sequences of the 3 adaptor molecules for the 3 internal standards are the following:
ARBCL30 (SEQ ID NO:20):
   5'-gtagcttaccctttagacctttttgaagaatctgttcccttacgactcgataagagctc t-3'
ACAB30 (SEQ ID NO:21):
   5'-ccaccacatctgctactgcagtgctgaatgactcttgctgaggctattcaaggcggcat c-3'
ARUBI30 (SEQ ID NO:22):
   5'-gacggcttctacattgcccctgctttcatgatacagttcaatcgccgagtctacgcggt a-3'
Three internal standards are added. The relative binding efficiency of both sets of capture molecules are calculated at the 3 scanner settings and used for correction of the target detection in both sets of capture molecules. The correction is performed at each scanner setting for gene which quantification is linear.

The part of the adaptor molecule that is complementary to the capture molecule is indicated in bold.

The adaptor molecules are obtained by chemical synthesis. For the rest, the experiment is performed as in example 1 and signals obtained on the internal standard capture molecules are used for correction of hybridization efficiency between the target-specific and target-non specific capture molecules.

## Claims

1. A detection and quantification method of a first set and a second set of target molecules (1, 3) being possibly present simultaneously in a biological sample upon a (micro)array solid support surface (6), said (micro)array (7) comprising a first set (A) and a second set (B) of capture molecules (2, 4), present in different locations (8, 9) of the solid support surface (6), wherein the first set of capture molecules (A) is made of at least 3 capture molecules (2) which are specific of the first set of target molecules (1) and fix directly the first set of target molecules (1) by specific molecular recognition, and wherein the second set of capture molecules (B) is made of at least 3 capture molecules (4) which are unrelated and have no direct binding affinity with the second set of target molecules (3), the second set of capture molecules (B) being able to fix the second set of target molecules (3) through adaptor molecules (5), and wherein a relative quantification of the first set of target molecules (1) compared to the second set of target molecules (3) is obtained by a correction factor calculated through the use of at least one identical molecule (x) simultaneously quantified on both sets (A, B) of capture molecules (2, 4).

2. The method according to claim 1, wherein the relative quantification of the target molecules belonging to a set are classified into at least 2 sub-sets of target molecules and are corrected according to different correction factors.

3. The method according to claim 1, wherein the target molecules belonging to a set are classified into at least 2 sub-sets of target molecules which are quantified by different settings of a detection scanner

4. The method according to claim 2, wherein the sub-sets of target molecules correspond to different concentrations of target molecules.

5. The method according to any of the preceding claims, wherein the detection of the target molecules (1, 3) belonging to the first set or the second set of target molecules are quantitative and wherein said quantitative detection is corrected by a correction factor that allows a comparable and simultaneous quantification of all target molecules initially present in the sample and fixed upon their corresponding capture molecules.

6. The method according to claim 5, wherein the quantitative detection is corrected by a detection of at least one standard molecule of known concentration added to the sample and submitted to the same detection step as the target molecules.

7. The method according to claim 5 wherein at least one target molecule is detected on both sets (A, B) of capture molecules (2, 4).

8. The method according to claim 6 or 7 wherein the fixation efficiency of target molecules (1, 3) on the two sets (A, B) of capture molecules (2, 4) is corrected by quantitative detection of target and/or standard molecules in both sets (A, B).

9. The method according to claim 1, wherein the capture molecules (4) of the second set (B) are totipotent capture molecules.

10. The method according to any of the preceding claims, wherein the detection is performed on at least two arrays (7', 7") present on the surface (6) of the solid support; wherein said at least two arrays (7', 7'') comprise capture molecules (2) of a first set (A) for the detection of the same target molecules (1); and
- each array comprises different sub sets (B, B') of capture molecules (4, 4') for the detection of target molecules (3, 3') that are different from one array (7') to another array (7").

11. The method according to any of the preceding claims, wherein the target, capture and adaptor molecules are nucleotide sequences.

12. The method according to the claim 11, wherein the fixing of the target molecules (3) upon the second sub set(s) (B) of capture molecules (4) is obtained by a sandwich hybridization between the capture molecules (4), the target molecules (3) through the adaptor molecules (5).

13. The method according to the claim 12, wherein the adaptor molecule (5) comprises a first portion (11), which allows an hybridization by complementary base pairing with a specific terminal portion of the capture molecule (4), and wherein the adaptor molecule (5) comprises a second portion (10) which is specific for at least a portion of one or more target molecules (3).

14. The method according to any of the claims 1 to 10, wherein the target molecules and capture molecules are proteins and wherein the adaptor molecules are chimeric proteins or hybrid antibodies.

15. The method according to any of the claims 1 to 10, wherein the target and the capture molecules are respectively:
- antigens and antibodies (or hypervariable portions of said antibodies) or
- antibodies (or hypervariable portions of said antibodies) and antigens.

16. The method according to any of the preceding claims, wherein the capture molecules (4) of the second set (B) comprise a terminal reactive chemical function able to bind specifically to adaptor molecules (5)

17. The method according to claim 16, wherein said terminal reactive chemical function is selected from the group consisting of aldehyde groups, epoxy groups, acrylate groups or is a mixture thereof.

18. A detection and quantification kit which comprises:
- a solid support having a surface (6) comprising at least two sets (A, B) of capture molecules (2, 4) present in different locations (8, 9) of said solid support surface (6),
- wherein the first set (A) of capture molecules at a first location (8), comprises at least three different capture molecules (2) being specific for a first set of target molecules (1) to be detected and quantified in a sample, said capture molecules being able to fix directly the target molecules (1),
- wherein the second set (B) of capture molecules at a second location (9) having at least three capture molecules (4) unrelated to a second set of target molecules (3) to be detected and quantified and having no direct binding affinity to fix target molecules (3), and
- one or more adaptor molecules (5) which allow the binding of the target molecules (3) of the second set onto the capture molecules (4) of the second set (B),
- possibly means and information for obtaining a relative quantification of the first set of target molecules (1) compared to the second set of target molecules (3) and
- wherein the capture molecules (4) of the second set (B) are totipotent capture molecules.

19. The detection and quantification kit according to claim 18, wherein means for obtaining the relative quantification is a standard molecule to be added in the sample with the target molecules (1, 3) and submitted to the same detection step.

20. The kit according to any of the preceding claims 18 to 19, wherein the solid support surface (6) comprises at least two arrays (7', 7") comprising capture molecules (2) of the first set (A) for the detection of the same target molecules (1) and each array (7', 7") comprising different sets (B, B') of capture molecules (4, 4') for the detection of target molecules (3, 3') being different from one array (7') to another array (7").

21. The kit according to any of the preceding claims 18 to 20, wherein target, capture and adaptor molecules are nucleotide sequences.

22. The kit according to any of the preceding claims 18 to 20, wherein target and capture molecules are proteins and wherein adaptor molecules are chimeric proteins or hybrid antibodies.

23. The kit according to any of the preceding claims 18 to 20, wherein targets and capture molecules are respectively :
- antigens and antibodies (or hypervariable portions of said antibodies); or
- antibodies (or hypervariable portions of said antibodies) and antigens.

24. The kit according to claim 21, wherein the binding of capture molecules (4) to target molecules (3) is obtained by sandwich hybridization between capture molecules (4), target molecules (3) and adaptor molecules (5).

25. The kit according to claim 24, wherein the adaptor molecule (5) comprises a first portion (11) which allows a hybridization by complementary base pairing with a specific terminal portion of the capture molecules (4) and wherein the adaptor molecule comprises a second portion (10) which is specific for at least a portion of one or more target molecules (3).

26. The kit according to any of the preceding claims 18 to 25, wherein the capture molecules (4) of the second set (B) comprise a terminal reactive chemical function able to bind specifically to the adaptor molecules (5).

27. The kit according to claim 26, wherein said terminal reactive chemical function is selected from the group consisting of an aldehyde group, an epoxy group and an acrylate group or is a mixture thereof.

## Patentansprüche

1. Nachweis- und Mengenbestimmungsverfahren für eine erste Gruppe und eine zweite Gruppe von Zielmolekülen (1, 3), welche in einer biologischen Probe möglicherweise gleichzeitig auf einer festen Trägerfläche (6) eines (Mikro-)Arrays vorliegen, wobei der (Mikro-) Array (7) eine erste Gruppe (A) und eine zweite Gruppe (B) von Einfangmolekülen (2, 4) umfasst, welche an verschiedenen Orten (8, 9) der festen Trägerfläche (6) vorliegen, wobei die erste Gruppe von Einfangmolekülen (A) aus mindestens 3 Einfangmolekülen (2) aufgebaut ist, welche für die erste Gruppe von Zielmolekülen (1) spezifisch sind und die erste Gruppe von Zielmolekülen (1) durch spezifische molekulare Erkennung direkt binden, und wobei die zweite Gruppe von Einfangmolekülen (B) aus mindestens 3 Einfangmolekülen (4) aufgebaut ist, welche keine Beziehung und keine direkte Bindungsaffinität zu der zweiten Gruppe von Zielmolekülen (3) aufweisen, wobei die zweite Gruppe von Einfangmolekülen (B) die zweite Gruppe von Zielmolekülen (3) über Adaptermoleküle (5) binden kann, und wobei eine relative Bestimmung der Menge der ersten Gruppe von Zielmolekülen (1) im Vergleich zu der zweiten Gruppe von Zielmolekülen (3) durch einen Korrekturfaktor erreicht werden kann, welcher durch die Verwendung zumindest eines identischen Moleküls (x) berechnet wird, dessen Menge gleichzeitig in beiden Gruppen (A, B) von Einfangmolekülen (2, 4) bestimmt wird.

2. Verfahren nach Anspruch 1, wobei die relative Bestimmung der Menge der Zielmoleküle, welche zu einer Gruppe gehören, in mindestens 2 Untergruppen von Zielmolekülen klassifiziert wird und mit verschiedenen Korrekturfaktoren berichtigt wird.

3. Verfahren nach Anspruch 1, wobei die Zielmoleküle, welche zu einer Gruppe gehören, in mindestens 2 Untergruppen von Zielmolekülen klassifiziert werden, deren Mengen durch verschiedene Einstellungen eines Messstellenabtasters für den Nachweis bestimmt werden.

4. Verfahren nach Anspruch 2, wobei die Untergruppen der Zielmoleküle verschiedenen Konzentrationen der Zielmoleküle entsprechen.

5. Verfahren nach einem der vorhergehenden Ansprüche, wobei der Nachweis der Zielmoleküle (1, 3), welche zu der ersten Gruppe oder der zweiten Gruppe von Zielmolekülen gehören, quantitativ ist, und wobei der quantitative Nachweis durch einen Korrekturfaktor berichtigt wird, welcher eine vergleichbare und gleichzeitige Bestimmung der Menge aller Zielmoleküle ermöglicht, die anfänglich in der Probe vorliegen und an ihren entsprechenden Einfangmolekülen gebunden sind.

6. Verfahren nach Anspruch 5, wobei der quantitative Nachweis durch einen Nachweis mindestens eines Standardmoleküls bekannter Konzentration berichtigt wird, welches der Probe zugegeben und demselben Nachweisschritt wie die Zielmoleküle unterzogen wird.

7. Verfahren nach Anspruch 5, wobei mindestens ein Zielmolekül in beiden Gruppen (A, B) der Einfangmoleküle (2, 4) nachgewiesen wird.

8. Verfahren nach Anspruch 6 oder 7, wobei die Effizienz der Bindung der Zielmoleküle (1, 3) an den beiden Gruppen (A, B) der Einfangmoleküle (2, 4) durch einen quantitativen Nachweis von Ziel- und/oder Standardmolekülen in beiden Gruppen (A, B) berichtigt wird.

9. Verfahren nach Anspruch 1, wobei die Einfangmoleküle (4) der zweiten Gruppe (B) totipotente Einfangmoleküle sind.

10. Verfahren nach einem der vorhergehenden Ansprüche, wobei der Nachweis auf mindestens zwei Arrays (7', 7") durchgeführt wird, welche auf der Fläche (6) des festen Trägers vorliegen; wobei die mindestens zwei Arrays (7', 7") Einfangmoleküle (2) einer ersten Gruppe (A) für den Nachweis derselben Zielmoleküle (1) umfassen; und jeder Array verschiedene Untergruppen (B, B') von Einfangmolekülen (4, 4') für den Nachweis von Zielmolekülen (3, 3') umfasst, welche sich von einem Array (7') zu einem anderen Array (7") unterscheiden.

11. Verfahren nach einem der vorhergehenden Ansprüche, wobei es sich bei den Ziel-, Einfang- und Adaptermolekülen um Nukleotidsequenzen handelt.

12. Verfahren nach Anspruch 11, wobei die Bindung der Zielmoleküle (3) an der/den zweiten Untergruppe(n) (B) der Einfangmoleküle (4) durch eine Sandwich-Hybridisierung zwischen den Einfangmolekülen (4), den Zielmolekülen (3) durch die Adaptermoleküle (5) erreicht wird.

13. Verfahren nach Anspruch 12, wobei das Adaptermolekül (5) einen ersten Abschnitt (11) umfasst, welcher eine Hybridisierung durch Komplementärbasenpaarung mit einem spezifischen Endabschnitt des Einfangmoleküls (4) ermöglicht, und wobei das Adaptermolekül (5) einen zweiten Abschnitt (10) umfasst, welcher für mindestens einen Abschnitt eines oder mehrerer Zielmoleküle (3) spezifisch ist.

14. Verfahren nach einem der Ansprüche 1 bis 10, wobei die Zielmoleküle und die Einfangmoleküle Proteine sind, und wobei die Adaptermoleküle chimäre Proteine oder hybride Antikörper sind.

15. Verfahren nach einem der Ansprüche 1 bis 10, wobei die Zielmoleküle und die Einfangmoleküle jeweils entsprechend
- Antigene und Antikörper (oder hypervariable Abschnitte der Antikörper) oder
- Antikörper (oder hypervariable Abschnitte der Antikörper) und Antigene
sind.

16. Verfahren nach einem der vorhergehenden Ansprüche, wobei die Einfangmoleküle (4) der zweiten Gruppe (B) eine endständige reaktive chemische Funktionalität umfassen, welche spezifisch an den Adaptermolekülen (5) binden kann.

17. Verfahren nach Anspruch 16, wobei die endständige reaktive chemische Funktionalität ausgewählt ist aus der Gruppe, bestehend aus Aldehydgruppen, Epoxygruppen, Acrylatgruppen oder einem Gemisch davon.

18. Nachweis- und Mengenbestimmungs-Bausatz, welcher das Folgende umfasst:
- einen festen Träger mit einer Fläche (6), welche mindestens zwei Gruppen (A, B) von Einfangmolekülen (2, 4) umfasst, die an verschiedenen Orten (8, 9) der festen Trägerfläche (6) vorliegen,
- wobei die erste Gruppe (A) von Einfangmolekülen an einem ersten Ort (8) mindestens drei verschiedene Einfangmoleküle (2) umfasst, welche für eine erste Gruppe von Zielmolekülen (1), welche in einer Probe nachzuweisen und mengenmäßig zu bestimmen sind, spezifisch sind, wobei die Einfangmoleküle die Zielmoleküle (1) direkt binden können,
- wobei die zweite Gruppe (B) von Einfangmolekülen an einem zweiten Ort (9) mindestens drei Einfangmoleküle (4) aufweist, welche keine Beziehung zu einer zweiten Gruppe von Zielmolekülen (3) aufweisen, welche nachzuweisen und mengenmäßig zu bestimmen sind, und keine direkte Bindungsaffinität zu den Zielmolekülen (3) aufweisen, und
- ein oder mehrere Adaptermoleküle (5), welche die Bindung der Zielmoleküle (3) der zweiten Gruppe an den Einfangmolekülen (4) der zweiten Gruppe (B) ermöglichen,
- gegebenenfalls Mittel und Daten zum Erreichen einer relativen Bestimmung der Menge der ersten Gruppe von Zielmolekülen (1) im Vergleich zu der zweiten Gruppe von Zielmolekülen (3), und
- wobei es sich bei den Einfangmoleküle (4) der zweiten Gruppe (B) um totipotente Einfangmoleküle handelt.

19. Nachweis- und Mengenbestimmungs-Bausatz nach Anspruch 18, wobei das Mittel zum Erreichen der relativen Mengenbestimmung ein Standardmolekül ist, welches der Probe mit den Zielmolekülen (1, 3) zuzugeben demselben Nachweisschritt zu unterziehen ist.

20. Bausatz nach einem der vorhergehenden Ansprüche 18 oder 19, wobei die feste Trägerfläche (6) mindestens zwei Arrays (7', 7") umfasst, welche Einfangmoleküle (2) der ersten Gruppe (A) für den Nachweis derselben Zielmoleküle (1) umfassen, und jeder Array (7', 7") verschiedene Gruppen (B, B') von Einfangmolekülen (4, 4') für den Nachweis von Zielmolekülen (3, 3') umfasst, welche sich von einem Array (7') zu einem anderen Array (7") unterscheiden.

21. Bausatz nach einem der vorhergehenden Ansprüche 18 bis 20, wobei es sich bei den Ziel-, Einfang- und Adaptermolekülen um Nukleotidsequenzen handelt.

22. Bausatz nach einem der vorhergehenden Ansprüche 18 bis 20, wobei die Zielmoleküle und die Einfangmoleküle Proteine sind, und wobei die Adaptermoleküle chimäre Proteine oder hybride Antikörper sind.

23. Bausatz nach einem der vorhergehenden Ansprüche 18 bis 20, wobei die Zielmoleküle und die Einfangmoleküle jeweils entsprechend
- Antigene und Antikörper (oder hypervariable Abschnitte der Antikörper) oder
- Antikörper (oder hypervariable Abschnitte der Antikörper) und Antigene
sind.

24. Bausatz nach Anspruch 21, wobei die Bindung der Einfangmoleküle (4) an den Zielmolekülen (3) durch eine Sandwich-Hybridisierung zwischen Einfangmolekülen (4), Zielmolekülen (3) und Adaptermolekülen (5) erreicht wird.

25. Bausatz nach Anspruch 24, wobei das Adaptermolekül (5) einen ersten Abschnitt (11) umfasst, welcher eine Hybridisierung durch Komplementärbasenpaarung mit einem spezifischen Endabschnitt der Einfangmoleküle (4) ermöglicht, und wobei das Adaptermolekül einen zweiten Abschnitt (10) umfasst, welcher für mindestens einen Abschnitt eines oder mehrerer Zielmoleküle (3) spezifisch ist.

26. Bausatz nach einem der vorhergehenden Ansprüche 18 bis 25, wobei die Einfangmoleküle (4) der zweiten Gruppe (B) eine endständige reaktive chemische Funktionalität umfassen, welche spezifisch an den Adaptermolekülen (5) binden kann.

27. Bausatz nach Anspruch 26, wobei die endständige reaktive chemische Funktionalität ausgewählt ist aus der Gruppe, bestehend aus einer Aldehydgruppe, einer Epoxygruppe, einer Acrylatgruppe oder einem Gemisch davon.

## Revendications

1. Procédé de détection et de quantification d'un premier ensemble et d'un second ensemble de molécules cibles (1, 3), qui sont éventuellement présents simultanément dans un échantillon biologique, sur une surface (6) du support solide d'un (micro) réseau, ledit (micro)réseau (7) comprenant un premier ensemble (A) et un second ensemble (B) de molécules de capture (2, 4) présents à des emplacements différents (8, 9) de la surface (6) du support solide, dans lequel le premier ensemble de molécules de capture (A) se compose d'au moins 3 molécules de capture (2) qui sont spécifiques au premier ensemble de molécules cibles (1) et fixent directement le premier ensemble de molécules cibles (1) par reconnaissance moléculaire spécifique, et dans lequel le second ensemble de molécules de capture (B) se compose d'au moins 3 molécules de capture (4) qui ne sont pas apparentées et qui n'ont pas d'affinité de liaison directe avec le second ensemble de molécules cibles (3), le second ensemble de molécules de capture (B) étant capable de fixer le second ensemble de molécules cibles (3) par des molécules adaptatrices (5), et dans lequel une quantification relative du premier ensemble de molécules cibles (1) en comparaison du second ensemble de molécules cibles (3) est obtenue par le biais d'un facteur de correction calculé en utilisant au moins une molécule identique (x) quantifiée simultanément sur les deux ensembles (A, B) de molécules de capture (2, 4).

2. Procédé selon la revendication 1, dans lequel la quantification relative des molécules cibles appartenant à un ensemble est classée en au moins deux sous-ensembles de molécules cibles et est corrigée selon différents facteurs de correction.

3. Procédé selon la revendication 1, dans lequel les molécules cibles appartenant à un ensemble sont classées en au moins deux sous-ensembles de molécules cibles qui sont quantifiées par différents paramètres d'un scanner de détection.

4. Procédé selon la revendication 2, dans lequel les sous-ensembles de molécules cibles correspondent à différentes concentrations de molécules cibles.

5. Procédé selon l'une quelconque des revendications précédentes, dans lequel la détection des molécules cibles (1, 3) appartenant au premier ensemble ou au second ensemble de molécules cibles est quantitative, et dans lequel ladite détection quantitative est corrigée par un facteur de correction qui permet une quantification comparable et simultanée de toutes les molécules cibles initialement présentes dans l'échantillon et fixées sur leurs molécules de capture correspondantes.

6. Procédé selon la revendication 5, dans lequel la détection quantitative est corrigée par une détection d'au moins une molécule standard de concentration connue, que l'on a ajoutée à l'échantillon et soumise à la même étape de détection que les molécules cibles.

7. Procédé selon la revendication 5 dans lequel au moins une molécule cible est détectée sur les deux ensembles (A, B) de molécules de capture (2, 4).

8. Procédé selon la revendication 6 ou 7, dans lequel l'efficacité de fixation des molécules cibles (1, 3) sur les deux ensembles (A, B) de molécules de capture (2, 4) est corrigée par une détection quantitative de molécules cibles et/ou standard dans les deux ensembles (A, B).

9. Procédé selon la revendication 1, dans lequel les molécules de capture (4) du second ensemble (B) sont des molécules de capture totipotentes.

10. Procédé selon l'une quelconque des revendications précédentes, dans lequel la détection est effectuée sur au moins deux réseaux (7', 7") présents sur la surface (6) du support solide; dans lequel lesdits au moins deux réseaux (7', 7") comprennent des molécules de capture (2) d'un premier ensemble (A) pour la détection des mêmes molécules cibles.(1); et
- chaque réseau comprend différents sous-ensembles (B, B') de molécules de capture (4, 4') pour la détection de molécules cibles (3, 3') qui sont différents d'un réseau (7') à l'autre (7").

11. Procédé selon l'une quelconque des revendications précédentes, dans lequel les molécules cibles, les molécules de capture et les molécules adaptatrices sont des séquences nucléotidiques.

12. Procédé selon la revendication 11, dans lequel la fixation des molécules cibles (3) sur les seconds sous-ensembles (B) de molécules de capture (4) est obtenue par une hybridation de type sandwich entre les molécules de capture (4) et les molécules cibles (3) par le biais des molécules adaptatrices (5).

13. Procédé selon la revendication 12, dans lequel la molécule adaptatrice (5) comprend une première partie (11) qui permet une hybridation par appariement de bases complémentaires avec une partie terminale spécifique de la molécule de capture (4), et dans lequel la molécule adaptatrice (5) comprend une seconde partie (10) qui est spécifique à au moins une partie d'une ou plusieurs molécules cibles (3).

14. Procédé selon l'une quelconque des revendications 1 à 10, dans lequel les molécules cibles et les molécules de capture sont des protéines et dans lequel les molécules adaptatrices sont des protéines chimères ou des anticorps hybrides.

15. Procédé selon l'une quelconque des revendications 1 à 10, dans lequel les molécules cibles et les molécules de capture sont respectivement :
- des antigènes et des anticorps (ou des parties hypervariables desdits anticorps), ou
- des anticorps (ou des parties hypervariables desdits anticorps) et des antigènes.

16. Procédé selon l'une quelconque des revendications précédentes, dans lequel les molécules de capture (4) du second ensemble (B) comprennent une fonction chimique réactive en position terminale capable de se lier spécifiquement aux molécules adaptatrices (5).

17. Procédé selon la revendication 16, dans lequel ladite fonction chimique réactive en position terminale est choisie dans le groupe constitué des groupements aldéhyde, des groupements époxy, des groupements acrylate ou est un mélange de ceux-ci.

18. Trousse de détection et de quantification qui comprend:
- un support solide ayant une surface (6) comprenant au moins deux ensembles (A, B) de molécules de capture (2, 4) présents à des emplacements différents (8, 9) de ladite surface (6) de support solide,
- où le premier ensemble (A) de molécules de capture à un premier emplacement (8) comprend au moins trois molécules de capture différentes (2) qui sont spécifiques à un premier ensemble de molécules cibles (1) que l'on souhaite détecter et quantifier dans un échantillon, lesdites molécules de capture étant capables de fixer directement les molécules cibles (1),
- où le second ensemble (B) de molécules de capture à un second emplacement (9) présente au moins trois molécules de capture (4) non apparentées à un second ensemble de molécules cibles (3) que l'on souhaite détecter et quantifier et ne présente pas d'affinité de liaison directe pour fixer les molécules cibles (3), et
- une ou plusieurs molécules adaptatrices (5) qui permettent la liaison des molécules cibles (3) du second ensemble avec les molécules de capture (4) du second ensemble (B),
- éventuellement des moyens et des informations pour obtenir une quantification relative du premier ensemble de molécules cibles (1) en comparaison du second ensemble de molécules cibles (3), et
- où les molécules de capture (4) du second ensemble (B) sont des molécules de capture totipotentés.

19. Trousse de détection et de quantification selon la revendication 18, dans laquelle les moyens pour obtenir une quantification relative sont une molécule standard que l'on doit ajouter à l'échantillon contenant les molécules cibles (1, 3) et soumettre à la même étape de détection.

20. Trousse selon l'une quelconque des revendications précédentes 18 et 19, dans laquelle la surface (6) du support solide comprend au moins deux réseaux (7', 7") comprenant des molécules de capture (2) du premier ensemble (A) pour la détection des mêmes molécules cibles (1) et chaque réseau (7', 7") comprenant différents ensembles (B, B') de molécules de capture (4, 4') pour la détection de molécules cibles (3, 3') différentes d'un réseau (7') à l'autre (7").

21. Trousse selon l'une quelconque des revendications précédentes 18 à 20, dans laquelle les molécules cibles, les molécules de capture et les molécules adaptatrices sont des séquences nucléotidiques.

22. Trousse selon l'une quelconque des revendications précédentes 18 à 20, dans laquelle les molécules cibles et les molécules de capture sont des protéines, et dans laquelle les molécules adaptatrices sont des protéines chimères ou des anticorps hybrides.

23. Trousse selon l'une quelconque des revendications précédentes 18 à 20, dans laquelle les molécules cibles et les molécules de capture sont respectivement:
- des antigènes et des anticorps (ou des parties hypervariables desdits anticorps), ou
- des anticorps (ou des parties hypervariables desdits anticorps) et des antigènes.

24. Trousse selon la revendication 21, dans laquelle la liaison des molécules de capture (4) aux molécules cibles (3) est obtenue par hybridation de type sandwich entre les molécules de capture (4), les molécules cibles (3) et les molécules adaptatrices (5).

25. Trousse selon la revendication 24, dans laquelle la molécule adaptatrice (5) comprend une première partie (11) qui permet une hybridation par appariement de bases complémentaires avec une partie terminale spécifique des molécules de capture (4), et dans laquelle la molécule adaptatrice comprend une seconde partie (10) qui est spécifique à au moins une partie d'une ou plusieurs molécules cibles (3).

26. Trousse selon l'une quelconque des revendications précédentes 18 à 25, dans laquelle les molécules de capture (4) du second ensemble (B) comprennent une fonction chimique réactive en position terminale capable de se lier spécifiquement aux molécules adaptatrices (5).

27. Trousse selon la revendication 26, dans laquelle ladite fonction chimique réactive en position terminale est choisie dans le groupe constitué d'un groupement aldéhyde, d'un groupement époxy et d'un groupement acrylate ou est un mélange de ceux-ci.


### SEQUENCE LISTING

<110> Eppendorf AG
<120> CUSTOMIZED MICRO-ARRAY CONSTRUCTION AND ITS USE FOR TARGET MOLECULE DETECTION
<130> BP.EAT.004A/EP
<140> EP04447112.6
   <141> 2004-05-04
<160> 22
<170> PatentIn version 3.2
<210> 1
   <211> 50
   <212> DNA
   <213> Artificial sequence
<220>
   <223> PALFAS1 sense primer
<400> 1
   gcatggatgt tgctctcgcg tagacgactg gatggctagt tactgctctg 50
<210> 2
   <211> 50
   <212> DNA
   <213> Artificial sequence
<220>
   <223> PALUCP21 sense primer
<400> 2
   agtacgtcga cagacttagt cctgaagctc gatggctagt tactgctctg 50
<210> 3
   <211> 50
   <212> DNA
   <213> Artificial sequence
<220>
   <223> PALL191 sense primer
<400> 3
   ggctgatcat gtactccaag gtttgttatc gatggctagt tactgctctg 50
<210> 4
   <211> 22
   <212> DNA
   <213> Artificial sequence
<220>
   <223> PAL2 antisense primer
<220>
   <221> misc_feature
   <222> (1)..(1)
   <223> aminated a
<400> 4
   atgagtttca agatttcaac ag 22
<210> 5
   <211> 220
   <212> DNA
   <213> Artificial sequence
<220>
   <223> TPALFAS capture molecule
<220>
   <221> misc_feature
   <222> (1)..(1)
   <223> aminated a
<400> 5
   atgagtttca agatttcaac atgagtttca agatttcaac agcttctgat gttttccttg 60
   aagagattaa gcccaaggag tttacatctt gattgtgttg ttcagcactt tgaacatggt 120
   tggtcactgg attggctaaa aattgaagtt cagagcagta actagccatc cagagcagta 180
   actagccatc cagtcgtcta cgcgagagca acatccatgc 220
<210> 6
   <211> 220
   <212> DNA
   <213> Artificial sequence
<220>
   <223> TPALUCP2 capture molecule
<220>
   <221> misc_feature
   <222> (1)..(1)
   <223> aminated a
<400> 6
   atgagtttca agatttcaac atgagtttca agatttcaac agcttctgat gttttccttg 60
   aagagattaa gcccaaggag tttacatctt gattgtgttg ttcagcactt tgaacatggt 120
   tggtcactgg attggctaaa aattgaagtt cagagcagta actagccatc cagagcagta 180
   actagccatc gagcttcagg actaagtctg tcgacgtact 220
<210> 7
   <211> 220
   <212> DNA
   <213> Artificial sequence
<220>
   <223> TPALL19 capture molecule
<220>
   <221> misc_feature
   <222> (1)..(1)
   <223> aminated a
<400> 7
   atgagtttca agatttcaac atgagtttca agatttcaac agcttctgat gttttccttg 60
   aagagattaa gcccaaggag tttacatctt gattgtgttg ttcagcactt tgaacatggt 120
   tggtcactgg attggctaaa aattgaagtt cagagcagta actagccatc cagagcagta 180
   actagccatc gataacaaac cttggagtac atgatcagcc 220
<210> 8
   <211> 60
   <212> DNA
   <213> Artificial sequence
<220>
   <223> AFAS30 adaptor molecule
<400> 8
   ataaagtttt gggctgctgt gtggcaatgc gcatggatgt tgctctcgcg tagacgactg 60
<210> 9
   <211> 60
   <212> DNA
   <213> Artificial sequence
<220>
   <223> AUCP230 adaptor molecule
<400> 9
   atgccattgt caactgtact gagctggtga agtacgtcga cagacttagt cctgaagctc 60
<210> 10
   <211> 60
   <212> DNA
   <213> Artificial sequence
<220>
   <223> AL1930 adaptor molecule
<400> 10
   cgtcctccgc tgtggtaaaa agaaggtgtg ggctgatcat gtactccaag gtttgttatc 60
<210> 11
   <211> 80
   <212> DNA
   <213> Artificial sequence
<220>
   <223> AFAS50 adaptor molecule
<400> 11
   ataaagtttt gggctgctgt gtggcaatgc agaggcaaag agaaggaact gcatggatgt 60
   tgctctcgcg tagacgactg 80
<210> 12
   <211> 80
   <212> DNA
   <213> Artificial sequence
<220>
   <223> AUCP250 adaptor molecule
<400> 12
   atgccattgt caactgtact gagctggtga cctatgacct catcaaagat agtacgtcga 60
   cagacttagt cctgaagctc 80
<210> 13
   <211> 80
   <212> DNA
   <213> Artificial sequence
<220>
   <223> AL1950 adaptor molecule
<400> 13
   cgtcctccgc tgtggtaaaa agaaggtgtg gttggacccc aatgaaacca ggctgatcat 60
   gtactccaag gtttgttatc 80
<210> 14
   <211> 50
   <212> DNA
   <213> Artificial sequence
<220>
   <223> PALRBCL1 sense primer
<400> 14
   tctgttccct tacgactcga taagagctct gatggctagt tactgctctg 50
<210> 15
   <211> 50
   <212> DNA
   <213> Artificial sequence
<220>
   <223> PALCAB1 sense primer
<400> 15
   actcttgctg aggctattca aggcggcatc gatggctagt tactgctctg 50
<210> 16
   <211> 50
   <212> DNA
   <213> Artificial sequence
<220>
   <223> PALRUBI1 sense primer
<400> 16
   atacagttca atcgccgagt ctacgcggta gatggctagt tactgctctg 50
<210> 17
   <211> 220
   <212> DNA
   <213> Artificial sequence
<220>
   <223> TPALRBCL capture molecule
<220>
   <221> misc_feature
   <222> (1)..(1)
   <223> aminated a
<400> 17
   atgagtttca agatttcaac atgagtttca agatttcaac agcttctgat gttttccttg 60
   aagagattaa gcccaaggag tttacatctt gattgtgttg ttcagcactt tgaacatggt 120
   tggtcactgg attggctaaa aattgaagtt cagagcagta actagccatc cagagcagta 180
   actagccatc agagctctta tcgagtcgta agggaacaga 220
<210> 18
   <211> 220
   <212> DNA
   <213> Artificial sequence
<220>
   <223> TPALCAB capture molecule
<220>
   <221> misc_feature
   <222> (1)..(1)
   <223> aminated a
<400> 18
   atgagtttca agatttcaac atgagtttca agatttcaac agcttctgat gttttccttg 60
   aagagattaa gcccaaggag tttacatctt gattgtgttg ttcagcactt tgaacatggt 120
   tggtcactgg attggctaaa aattgaagtt cagagcagta actagccatc cagagcagta 180
   actagccatc gatgccgcct tgaatagcct cagcaagagt 220
<210> 19
   <211> 220
   <212> DNA
   <213> Artificial sequence
<220>
   <223> TPALRUBI capture molecule
<220>
   <221> misc_feature
   <222> (1)..(1)
   <223> aminated a
<400> 19
   atgagtttca agatttcaac atgagtttca agatttcaac agcttctgat gttttccttg 60
   aagagattaa gcccaaggag tttacatctt gattgtgttg ttcagcactt tgaacatggt 120
   tggtcactgg attggctaaa aattgaagtt cagagcagta actagccatc cagagcagta 180
   actagccatc taccgcgtag actcggcgat tgaactgtat 220
<210> 20
   <211> 60
   <212> DNA
   <213> Artificial sequence
<220>
   <223> ARBCL 30 adaptor molecule
<400> 20
   gtagcttacc ctttagacct ttttgaagaa tctgttccct tacgactcga taagagctct 60
<210> 21
   <211> 60
   <212> DNA
   <213> Artificial sequence
<220>
   <223> ACAB30 adaptor molecule
<400> 21
   ccaccacatc tgctactgca gtgctgaatg actcttgctg aggctattca aggcggcatc 60
<210> 22
   <211> 60
   <212> DNA
   <213> Artificial sequence
<220>
   <223> ARUBI30 adaptor molecule
<400> 22
   gacggcttct acattgcccc tgctttcatg atacagttca atcgccgagt ctacgcggta 60

### SEQUENCE LISTING

<110> Eppendorf AG
<120> CUSTOMIZED MICRO-ARRAY CONSTRUCTION AND ITS USE FOR TARGET MOLECULE DETECTION
<130> BP.EAT.004A/EP
<140> EP04447112.6
   <141> 2004-05-04
<160> 22
<170> PatentIn version 3.2
<210> 1
   <211> 50
   <212> DNA
   <213> Artificial sequence
<220>
   <223> PALFAS1 sense primer
<400> 1
   gcatggatgt tgctctcgcg tagacgactg gatggctagt tactgctctg 50
<210> 2
   <211> 50
   <212> DNA
   <213> Artificial sequence
<220>
   <223> PALUCP21 sense primer
<400> 2
   agtacgtcga cagacttagt cctgaagctc gatggctagt tactgctctg 50
<210> 3
   <211> 50
   <212> DNA
   <213> Artificial sequence
<220>
   <223> PALL191 sense primer
<400> 3
   ggctgatcat gtactccaag gtttgttatc gatggctagt tactgctctg 50
<210> 4
   <211> 22
   <212> DNA
   <213> Artificial sequence
<220>
   <223> PAL2 antisense primer
<220>
   <221> misc_feature
   <222> (1)..(1)
   <223> aminated a
<400> 4
   atgagtttca agatttcaac ag 22
<210> 5
   <211> 220
   <212> DNA
   <213> Artificial sequence
<220>
   <223> TPALFAS capture molecule
<220>
   <221> misc_feature
   <222> (1)..(1)
   <223> aminated a
<400> 5
   atgagtttca agatttcaac atgagtttca agatttcaac agcttctgat gttttccttg 60
   aagagattaa gcccaaggag tttacatctt gattgtgttg ttcagcactt tgaacatggt 120
   tggtcactgg attggctaaa aattgaagtt cagagcagta actagccatc cagagcagta 180
   actagccatc cagtcgtcta cgcgagagca acatccatgc 220
<210> 6
   <211> 220
   <212> DNA
   <213> Artificial sequence
<220>
   <223> TPALUCP2 capture molecule
<220>
   <221> misc_feature
   <222> (1)..(1)
   <223> aminated a
<400> 6
   atgagtttca agatttcaac atgagtttca agatttcaac agcttctgat gttttccttg 60
   aagagattaa gcccaaggag tttacatctt gattgtgttg ttcagcactt tgaacatggt 120
   tggtcactgg attggctaaa aattgaagtt cagagcagta actagccatc cagagcagta 180
   actagccatc gagcttcagg actaagtctg tcgacgtact 220
<210> 7
   <211> 220
   <212> DNA
   <213> Artificial sequence
<220>
   <223> TPALL19 capture molecule
<220>
   <221> misc_feature
   <222> (1) .. (1)
   <223> aminated a
<400> 7
   atgagtttca agatttcaac atgagtttca agatttcaac agcttctgat gttttccttg 60
   aagagattaa gcccaaggag tttacatctt gattgtgttg ttcagcactt tgaacatggt 120
   tggtcactgg attggctaaa aattgaagtt cagagcagta actagccatc cagagcagta 180
   actagccatc gataacaaac cttggagtac atgatcagcc 220
<210> 8
   <211> 60
   <212> DNA
   <213> Artificial sequence
<220>
   <223> AFAS30 adaptor molecule
<400> 8
   ataaagtttt gggctgctgt gtggcaatgc gcatggatgt tgctctcgcg tagacgactg 60
<210> 9
   <211> 60
   <212> DNA
   <213> Artificial sequence
<220>
   <223> AUCP230 adaptor molecule
<400> 9
   atgccattgt caactgtact gagctggtga agtacgtcga cagacttagt cctgaagctc 60
<210> 10
   <211> 60
   <212> DNA
   <213> Artificial sequence
<220>
   <223> AL1930 adaptor molecule
<400> 10
   cgtcctccgc tgtggtaaaa agaaggtgtg ggctgatcat gtactccaag gtttgttatc 60
<210> 11
   <211> 80
   <212> DNA
   <213> Artificial sequence
<220>
   <223> AFAS50 adaptor molecule
<400> 11
   ataaagtttt gggctgctgt gtggcaatgc agaggcaaag agaaggaact gcatggatgt 60
   tgctctcgcg tagacgactg 80
<210> 12
   <211> 80
   <212> DNA
   <213> Artificial sequence
<220>
   <223> AUCP250 adaptor molecule
<400> 12
   atgccattgt caactgtact gagctggtga cctatgacct catcaaagat agtacgtcga 60
   cagacttagt cctgaagctc 80
<210> 13
   <211> 80
   <212> DNA
   <213> Artificial sequence
<220>
   <223> AL1950 adaptor molecule
<400> 13
   cgtcctccgc tgtggtaaaa agaaggtgtg gttggacccc aatgaaacca ggctgatcat 60
   gtactccaag gtttgttatc 80
<210> 14
   <211> 50
   <212> DNA
   <213> Artificial sequence
<220>
   <223> PALRBCL1 sense primer
<400> 14
   tctgttccct tacgactcga taagagctct gatggctagt tactgctctg 50
<210> 15
   <211> 50
   <212> DNA
   <213> Artificial sequence
<220>
   <223> PALCAB1 sense primer
<400> 15
   actcttgctg aggctattca aggcggcatc gatggctagt tactgctctg 50
<210> 16
   <211> 50
   <212> DNA
   <213> Artificial sequence
<220>
   <223> PALRUBI1 sense primer
<400> 16
   atacagttca atcgccgagt ctacgcggta gatggctagt tactgctctg 50
<210> 17
   <211> 220
   <212> DNA
   <213> Artificial sequence
<220>
   <223> TPALRBCL capture molecule
<220>
   <221> misc_feature
   <222> (1)..(1)
   <223> aminated a
<400> 17
   atgagtttca agatttcaac atgagtttca agatttcaac agcttctgat gttttccttg 60
   aagagattaa gcccaaggag tttacatctt gattgtgttg ttcagcactt tgaacatggt 120
   tggtcactgg attggctaaa aattgaagtt cagagcagta actagccatc cagagcagta 180
   actagccatc agagctctta tcgagtcgta agggaacaga 220
<210> 18
   <211> 220
   <212> DNA
   <213> Artificial sequence
<220>
   <223> TPALCAB capture molecule
<220>
   <221> misc_feature
   <222> (1) .. (1)
   <223> aminated a
<400> 18
   atgagtttca agatttcaac atgagtttca agatttcaac agcttctgat gttttccttg 60
   aagagattaa gcccaaggag tttacatctt gattgtgttg ttcagcactt tgaacatggt 120
   tggtcactgg attggctaaa aattgaagtt cagagcagta actagccatc cagagcagta 180
   actagccatc gatgccgcct tgaatagcct cagcaagagt 220
<210> 19
   <211> 220
   <212> DNA
   <213> Artificial sequence
<220>
   <223> TPALRUBI capture molecule
<220>
   <221> misc_feature
   <222> (1)..(1)
   <223> aminated a
<400> 19
   atgagtttca agatttcaac atgagtttca agatttcaac agcttctgat gttttccttg 60
   aagagattaa gcccaaggag tttacatctt gattgtgttg ttcagcactt tgaacatggt 120
   tggtcactgg attggctaaa aattgaagtt cagagcagta actagccatc cagagcagta 180
   actagccatc taccgcgtag actcggcgat tgaactgtat 220
<210> 20
   <211> 60
   <212> DNA
   <213> Artificial sequence
<220>
   <223> ARBCL30 adaptor molecule
<400> 20
   gtagcttacc ctttagacct ttttgaagaa tctgttccct tacgactcga taagagctct 60
<210> 21
   <211> 60
   <212> DNA
   <213> Artificial sequence
<220>
   <223> ACAB30 adaptor molecule
<400> 21
   ccaccacatc tgctactgca gtgctgaatg actcttgctg aggctattca aggcggcatc 60
<210> 22
   <211> 60
   <212> DNA
   <213> Artificial sequence
<220>
   <223> ARUBI30 adaptor molecule
<400> 22
   gacggcttct acattgcccc tgctttcatg atacagttca atcgccgagt ctacgcggta 60